# EUROPEAN PATENT APPLICATION

(11) **EP 4 715 036 A1**
(43) Date of publication of application: **25.03.2026**
(21) Application number: 24811152.8
(22) Date of filing: 22.05.2024
(51) Int. Cl.: C12M 1/26

(54) **PIPETTE FOR SUCTIONING/HOLDING CELLS, AND INSTRUMENT FOR COLLECTING CELLS FROM FERTILIZED EGG OR EMBRYO**

(30) Priority: 23.05.2023 JP 2023084874
(71) Applicant: Kitazato Corporation, Fuji-shi, Shizuoka 416-0932 (JP)
(72) Inventor: INOUE, Futoshi, Fuji-shi, Shizuoka 416-0932 (JP); UCHIYAMA, Kazuo, Fuji-shi, Shizuoka 416-0932 (JP); OKITSU, Osamu, Tokyo 103-0014 (JP)
(74) Representative: Casalonga
(86) International application number: PCT/JP2024/018881
(87) International publication number: WO 2024/242146

(57) **Abstract**

A cell suction holding pipette 1 includes a hollow body 2 and a lumen 3 having a leading end opening 31 and a rear end opening 32. The hollow body 2 includes a leading end portion 21, and the leading end portion 21 includes an inclined surface portion 4 formed on an outer surface of the leading end portion 21 and inclining toward a leading end side extension line of a central axis of the lumen 3.

## Description

### Technical Field

The present invention relates to a cell suction holding pipette used at the time of collecting cells for preimplantation genetic testing for aneuploidy or intracytoplasmic sperm injection.

### Background Art

In recent years, preimplantation genetic testing for aneuploidy (PGT-A) in which some cells are collected to analyze genes is performed as a method for testing chromosomal abnormalities in fertilized eggs and embryos.

In PGT-A, after a fertilized egg has been cultured for two or three days and cleaved into four to eight blastomeres, or after a fertilized egg has been cultured for four to seven days, a blastocyst or one to three blastomeres are collected and examined for aneuploidy of the fertilized egg. Then, only a normal fertilized egg is transplanted into the maternal uterus, and therefore, it is thought that a miscarriage rate can be reduced and a pregnancy rate can be increased.

In order to perform PGT-A, it is necessary to hold the blastocyst or blastomeres thereof with a suction pipette, and to collect some (about five to ten cells) of trophectoderm cells from the held blastocyst or blastomeres. In the above-mentioned cell collecting operation, it is necessary to securely hold the blastocyst or blastomeres.

Also, in the field of biotechnology, injection of sperms and sperm cells into oocytes, and injection of nuclei, cytoplasm, and DNAs into cells are carried out under microscopic observation. A micromanipulation system is used for these operations.

In ICSI (Intracytoplasmic Sperm Injection), an oocyte is pierced with a microneedle (a micropipette with a blade edge) using a micromanipulator while the oocyte is suctioned and held by a suction pipette to perform a genetic modification operation, an intracytoplasmic sperm injection operation, or the like on the oocyte. In these operations as well, it is necessary to securely hold the oocyte.

A common suction pipette is shown in FIG. 11 of JP H9-168382A (Patent Document 1). The pipette shown in FIG. 11 of Patent Document 1 is constituted by a pipette body 4 that is a glass tube having a suction port 2 which is open to the outside and holds an operation target 1 such as a cell by suction, a connection port which is open to the outside and is used to perform suction under negative pressure, and an internal passage 3 connecting the suction port 2 and the connection port.

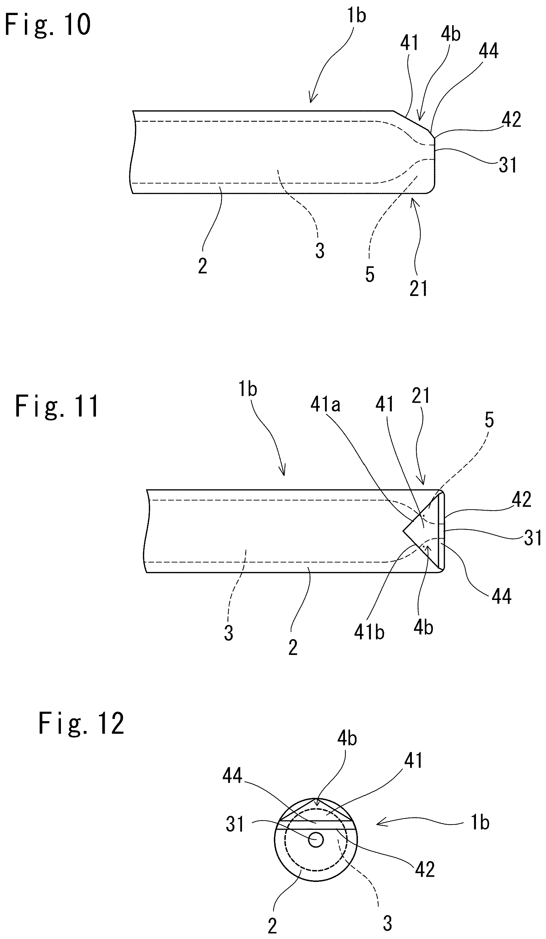

The connection port side portion of the pipette body 4 is connected to, for example, an injector (configured like a syringe) and the inside of the internal passage 3 is suctioned to generate negative pressure via the connection port by operating the injector. As a result, the operation target 1 such as a cell is suctioned and held by the suction port 2 as shown in FIG. 11. In this state, various operations are carried out. For example, if the operation target 1 is an oocyte, a hollow microneedle (a micropipette with a blade edge) 5 is inserted into the oocyte to inject a sperm and fertilize the oocyte. If the operation target 1 is any of various cells, the operation target 1 is pierced with the needle 5 or the like to form a hole therein. After these operations are finished, the injector is operated to generate positive pressure in the internal passage 3 via the connection port, thereby releasing the operation target 1 suctioned and held by the suction port 2.

A pipette shown in FIG. 1 of Patent Document 1 includes, in the vicinity of a suction port 22 for holding an operation target 1 (for example, an oocyte) by suction, a surrounding wall 25a that surrounds the operation target 1 suctioned and held by the suction port 22 from a lateral direction with respect to the direction in which the operation target 1 is suctioned via the suction port 22.

### Citation List

### Patent Document

Patent Document 1: JP H9-168382A

### Summary of Invention

### Technical Problem

In the case of the suction pipette shown in FIG. 11 of Patent Document 1, in order to suction and hold cells (e.g., an oocyte or blastocyst), it is necessary to suction the cells into another collection pipette, then discharge the cells above an outer surface of a leading end portion of the suction pipette, and guide the cells to a leading end surface of the suction pipette to suction and hold the cells. However, the cells discharged above the outer surface of the leading end portion of the suction pipette may fall to a side of the suction pipette, making it difficult to suction and hold the cells.

In the case of the suction pipette shown in FIG. 1 of Patent Document 1, it is possible to suction and hold cells with the suction pipette by discharging the cells in the vicinity of a leading end opening of the surrounding wall 25a. However, there is a distance between the leading end opening of the surrounding wall 25a and a leading end opening of the pipette body, and therefore, the cells need to be suctioned strongly, and this may cause damage on the cells. In addition, cells held as shown in FIG. 1 of Patent Document 1 may be pressed by the surrounding wall 25a, and this may cause damage on the cells.

An object of the present invention is to provide a cell suction holding pipette that facilitates a cell suctioning operation performed using the suction pipette and is unlikely to damage cells.

Also, in order to examine for chromosomal abnormalities in a fertilized egg or embryo, the fertilized egg or embryo is held using a suction pipette, and part of cells (specifically, part of trophectoderm cells) is collected from the held fertilized egg or embryo. This collecting operation is performed by bringing a leading end portion of a cell collecting pipette into contact with the fertilized egg or embryo in a state where the fertilized egg or embryo is held by the suction holding pipette, collecting part of the cells from the fertilized egg or embryo by suction, and then separating the suctioned cells from the fertilized egg or embryo.

An object of the present invention is to provide a device for collecting cells from a fertilized egg or embryo, which can facilitate the operation for separating suctioned cells from the fertilized egg or embryo.

### Solution to Problem

The above object is achieved by the following.

A cell suction holding pipette including a hollow body and a lumen having a leading end opening and a rear end opening, wherein the hollow body includes a leading end portion, and the leading end portion includes an inclined surface portion formed on an outer surface of the leading end portion and inclining toward a leading end side extension line of a central axis of the lumen.

The above object is achieved by the following.

A device for collecting cells from a fertilized egg or an embryo including the cell suction holding pipette described above and a cell suction collecting pipette, wherein the cell suction collecting pipette is a hollow tubular member including a leading end portion whose outer diameter is smaller than an outer diameter of a leading end portion of the cell suction holding pipette, the cell suction collecting pipette includes a hollow body, a leading end opening, a base end opening, and a lumen formed in the hollow body, and a leading end side portion of the lumen serves as a cell collecting portion.

### Brief Description of Drawings

FIG. 1 is a front view of a cell suction holding pipette of an embodiment of the present invention.
FIG. 2 is a plan view of the cell suction holding pipette shown in FIG. 1.
FIG. 3 is a longitudinal cross-sectional view of the cell suction holding pipette shown in FIG. 1.
FIG. 4 is an enlarged front view of a leading end portion of the cell suction holding pipette shown in FIG. 1.
FIG. 5 is an enlarged plan view of the leading end portion of the cell suction holding pipette shown in FIG. 2.
FIG. 6 is an enlarged right side view of the cell suction holding pipette shown in FIG. 1.
FIG. 7 is an enlarged front view of a leading end portion of a cell suction holding pipette according to another embodiment of the present invention.
FIG. 8 is a plan view of the cell suction holding pipette shown in FIG. 7.
FIG. 9 is a right side view of the cell suction holding pipette shown in FIG. 7.
FIG. 10 is an enlarged front view of a leading end portion of a cell suction holding pipette according to another embodiment of the present invention.
FIG. 11 is a plan view of the cell suction holding pipette shown in FIG. 10.
FIG. 12 is a right side view of the cell suction holding pipette shown in FIG. 10.
FIG. 13 is an enlarged front view of a leading end portion of a cell suction holding pipette according to another embodiment of the present invention.
FIG. 14 is a bottom view of the cell suction holding pipette shown in FIG. 13.
FIG. 15 is a right side view of the cell suction holding pipette shown in FIG. 13.
FIG. 16 is an enlarged right side view of a leading end portion of a cell suction holding pipette according to another embodiment of the present invention.
FIG. 17 is a front view of a cell suction holding pipette according to another embodiment of the present invention.
FIG. 18 is an explanatory diagram illustrating a cell suction holding operation by a cell suction holding pipette of the present invention.
FIG. 19 is an explanatory diagram illustrating the cell suction holding operation by the cell suction holding pipette of the present invention.
FIG. 20 is an explanatory diagram illustrating the cell suction holding operation by the cell suction holding pipette of the present invention.
FIG. 21 is a front view of a cell suction holding pipette according to another embodiment of the present invention.
FIG. 22 is an enlarged longitudinal cross-sectional view of a leading end portion of the cell suction holding pipette shown in FIG. 21.
FIG. 23 is an enlarged plan view of the leading end portion of the cell suction holding pipette shown in FIG. 21.
FIG. 24 is an enlarged right side view of the cell suction holding pipette shown in FIG. 21.
FIG. 25 is a front view of an embodiment of a cell suction collecting pipette used in a device for collecting cells from a fertilized egg or embryo according to the present invention.
FIG. 26 is a longitudinal cross-sectional view of the cell suction collecting pipette shown in FIG. 25.
FIG. 27 is an explanatory diagram illustrating a cell suction collecting operation by the device for collecting cells from a fertilized egg or embryo according to the present invention.
FIG. 28 is an explanatory diagram illustrating the cell suction collecting operation by the device for collecting cells from a fertilized egg or embryo according to the present invention.
FIG. 29 is an explanatory diagram illustrating the cell suction collecting operation by the device for collecting cells from a fertilized egg or embryo according to the present invention.
FIG. 30 is an explanatory diagram illustrating the cell suction collecting operation by the device for collecting cells from a fertilized egg or embryo according to the present invention.
FIG. 31 is an explanatory diagram illustrating the cell suction collecting operation by the device for collecting cells from a fertilized egg or embryo according to the present invention.

### Description of Embodiments

The following describes a cell suction holding pipette of the present invention with reference to embodiments shown in the drawings.

A cell suction holding pipette 1 of the present invention includes a hollow body 2 and a lumen 3 having a leading end opening 31 and a rear end opening 32. The hollow body 2 includes a leading end portion 21, and the leading end portion 21 includes an inclined surface portion 4 that is formed on an outer surface of the leading end portion 21 and inclines toward the leading end side extension line of a central axis of the lumen 3.

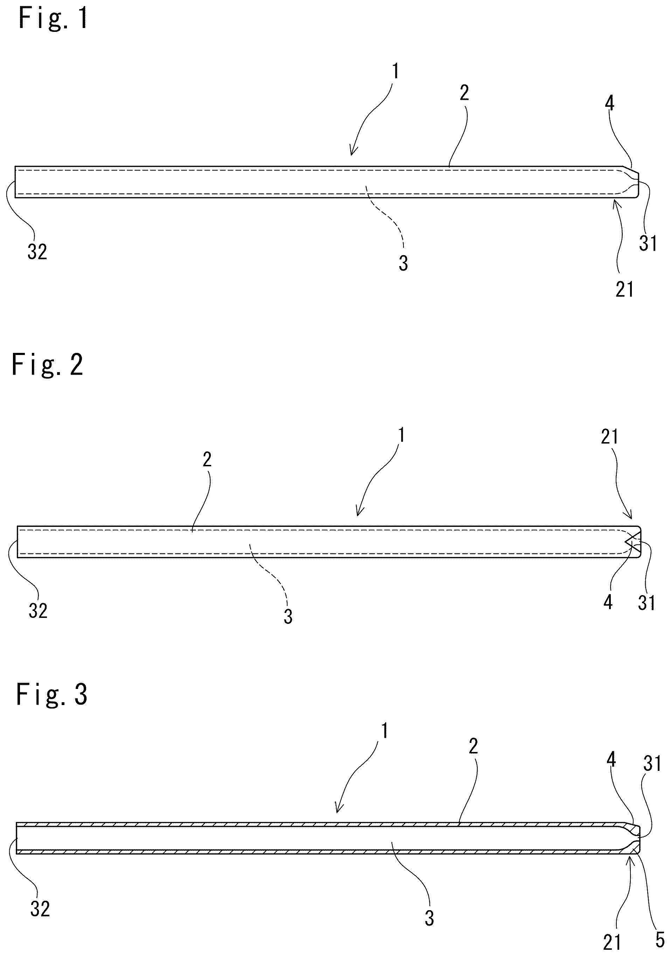

The cell suction holding pipette 1 according to the embodiment shown in FIGS. 1 to 6 is a hollow tubular member including the hollow body 2 and the lumen 3 formed in the hollow body.

In this embodiment, a straight tube having substantially the same outer diameter and extending linearly is used as the hollow body 2. The material of the hollow body 2 is glass or a hard synthetic resin, and is preferably transparent. Examples of the glass include glass containing silicate as a main component (silicate glass). Examples of silicate glass include soda lime glass, borosilicate glass, and quartz glass. The glass may contain components other than silicate as a main component. Examples of such glass include acrylic glass, chalcogenide glass, metallic glass, and organic glass. In particular, borosilicate glass is preferable because alkaline elution can be suppressed. Examples of the hard synthetic resin include polyolefins such as high-density polyethylene (HDPE) and polypropylene (PP), polystyrene, polyethylene terephthalate, and polycarbonate.

In the cell suction holding pipette 1 according to this embodiment, the lumen 3 includes a leading end small inner diameter portion, and the leading end portion 21 of the hollow body 2 includes a thick leading end portion 5. Therefore, the inner diameter of the lumen 3 in the hollow body 2 is not constant, but becomes smaller at the leading end portion.

More specifically, as shown in FIGS. 1, 2, and 5, in the cell suction holding pipette 1, the inner diameter of the lumen 3 sharply decreases in the leading end portion 21, and the leading end portion of the lumen 3 includes a diameter reduced portion and a portion that is short and has substantially the same inner diameter at the leading end of the diameter reduced portion. Accordingly, the leading end portion 21 of the cell suction holding pipette 1 includes the thick portion 5 having an annular shape. The inner diameter (opening diameter) of the leading end opening 31 of the lumen 3 is smaller than the inner diameter (opening diameter) of the rear end opening 32.

The outer diameter of the hollow body 2 (outer diameter of the leading end of the hollow body) of the cell suction holding pipette 1 is preferably 70 to 140 µm, and particularly preferably 80 to 130 µm. The inner diameter (opening diameter) of the leading end opening 31 is preferably 10 to 40 µm, and particularly preferably 12 to 35 µm.

Furthermore, when the outer diameter of the hollow body 2 (outer diameter of the leading end of the hollow body) of the cell suction holding pipette 1 is 70 to 110 µm, the inner diameter (opening diameter) of the leading end opening 31 is preferably 10 to 20 µm, and particularly preferably 12 to 18 µm. When the outer diameter of the hollow body 2 (outer diameter of the leading end of the hollow body) of the cell suction holding pipette 1 is 110 to 140 µm, the inner diameter (opening diameter) of the leading end opening 31 is preferably 20 to 40 µm, and particularly preferably 25 to 35 µm.

The cell suction holding pipette 1 of the present invention includes the inclined surface portion 4 that is formed on the outer surface of the leading end portion 21 of the hollow body 2 and inclines toward the leading end side extension line of the central axis of the lumen 3. As described above, the lumen 3 includes the leading end small inner diameter portion, and the leading end portion 21 of the hollow body 2 includes the thick leading end portion 5. Accordingly, in this embodiment, the inclined surface portion 4 is formed on the outer surface of the thick leading end portion 5. Therefore, the leading end portion 21 of the hollow body 2 is not weakened due to the inclined surface portion 4, and has sufficient strength.

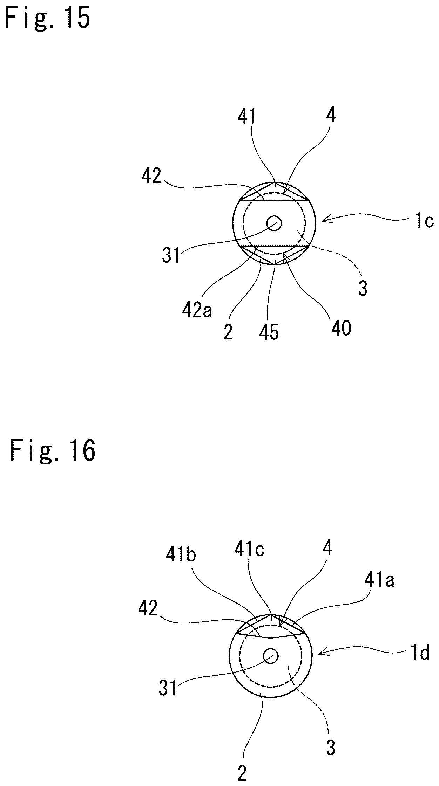

In the cell suction holding pipette 1 of this embodiment, the inclined surface portion 4 includes a planar inclined surface 41 as shown in FIGS. 2, 5, and 6. The inclined surface portion is preferably a flat surface. However, the planar inclined surface of the inclined surface portion may have a shape whose central portion is recessed as in the case of a planar inclined surface 41c of a cell suction holding pipette 1d of an embodiment shown in FIG. 16. Examples of the case where the planar inclined surface 41 has a shape whose central portion is recessed include a case where the planar inclined surface 41 slopes toward the center from both sides 41a and 41b and a case where the planar inclined surface 41 is gently curved inward.

In this embodiment, the planar inclined surface 41 is a substantially triangular inclined surface 41 extending to the leading end of the hollow body 2 with its width increasing toward the leading end. Preferably, the sides 41a and 41b of the planar inclined surface 41 have no edge, and are chamfered or rounded, for example. An axial length of the inclined surface portion 4 is preferably 40 to 160 µm, and particularly preferably 60 to 140 µm.

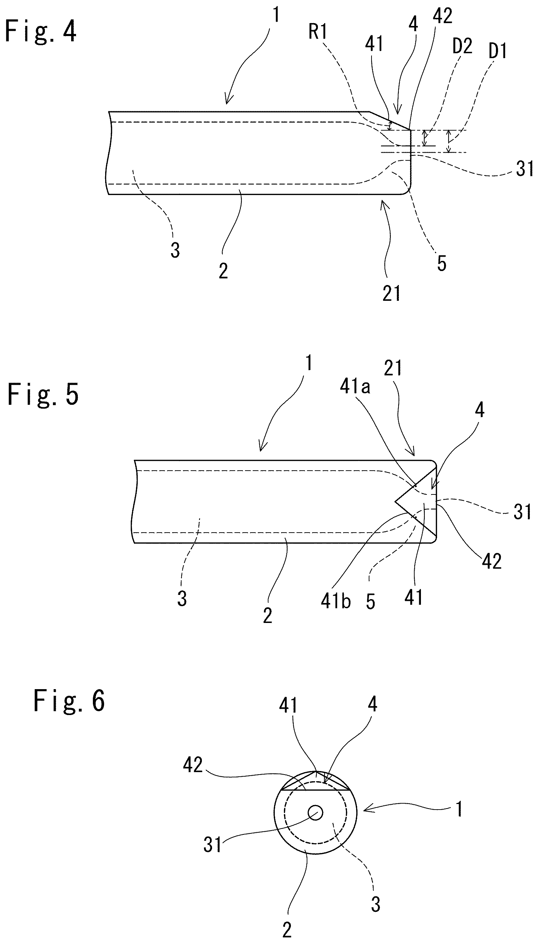

In the cell suction holding pipette 1 according to this embodiment, the planar inclined surface 41 reaches the leading end of the hollow body 2 and inclines at approximately the same angle as shown in FIGS. 1 and 4. The inclination angle (R1 in FIG. 4) of the planar inclined surface 41 with respect to the central axis of the lumen 3 is preferably 10 to 50 degrees. If the inclination angle is 10 degrees or more, it is possible to guide cells placed on the inclined surface portion 4 toward the leading end of the pipette, and if the inclination angle is 50 degrees or less, it is possible to restrict rapid movement of cells placed on the inclined surface portion 4. The inclination angle is particularly preferably 15 to 45 degrees. The inclination angle is more preferably 18 to 40 degrees. Also, the inclination angle (R1 in FIG. 4) of the planar inclined surface 41 with respect to the central axis of the lumen 3 may be 5 to 30 degrees. If the inclination angle is 5 degrees or more, it is possible to slowly guide cells placed on the inclined surface portion 4 toward the leading end of the pipette. The inclination angle is particularly preferably 5 to 25 degrees.

Also, a distance (D1 in FIG. 4) between a central portion at the leading end 42 of the inclined surface portion 4 and a central portion of the lumen 3 is preferably 10 to 40 µm. The distance is particularly preferably 15 to 35 µm. Also, a distance (D2 in FIG. 4) between the central portion at the leading end 42 of the inclined surface portion 4 and an upper end of the lumen 3 is preferably 7 to 35 µm. The distance is particularly preferably 10 to 30 µm.

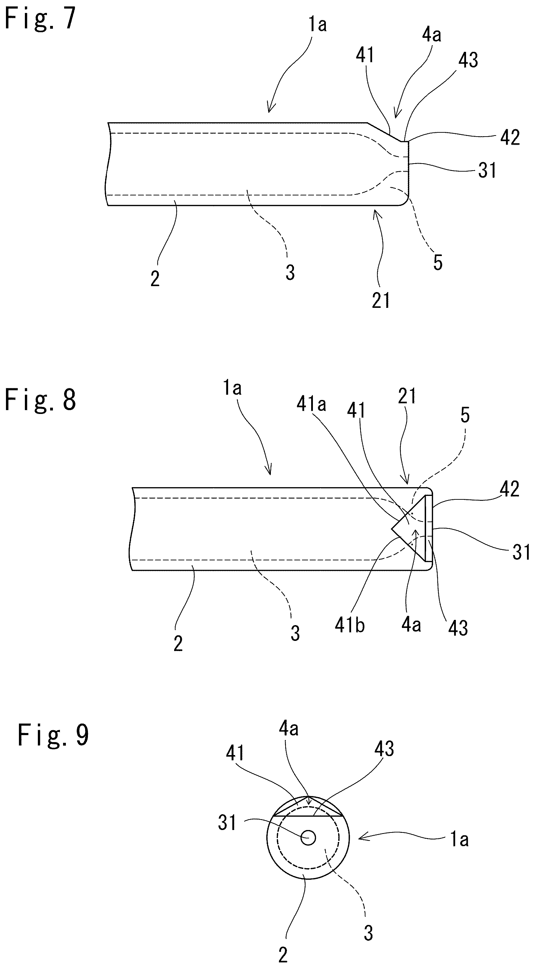

The cell suction holding pipette according to the present invention may also include an inclined surface portion 4a shown in FIGS. 7 to 9. As shown in FIGS. 7 to 9, in a cell suction holding pipette 1a of this embodiment, the inclined surface portion 4a includes a substantially triangular inclined surface (planar inclined surface) 41 whose width increases toward the leading end of the hollow body, and further includes a flat leading end portion 43 that is formed at the leading end of the inclined surface portion 4a and extends substantially in parallel with the central axis of the lumen 3. Specifically, the inclined surface portion 4a includes the substantially triangular inclined surface 41 whose width increases toward the leading end of the hollow body, and the flat leading end portion 43 extending from the leading end of the inclined surface 41 toward the leading end of the hollow body substantially in parallel with the central axis of the lumen 3, and the flat leading end portion 43 reaches the leading end 42 of the pipette. The only difference between the cell suction holding pipette 1a of this embodiment and the cell suction holding pipette 1 of the above embodiment is the difference in the shape of the inclined surface portion.

The axial length of the inclined surface portion 4a is preferably 40 to 160 µm, and particularly preferably 60 to 140 µm. The width (width in a direction orthogonal to the central axis of the pipette) of the flat leading end portion 43 is preferably 10 to 30 µm. The width is particularly preferably 15 to 25 µm.

As in the pipette 1 described above, in the cell suction holding pipette 1a of this embodiment as well, the inclination angle of the planar inclined surface 41 with respect to the central axis of the lumen 3 is preferably 10 to 50 degrees. The inclination angle is particularly preferably 15 to 45 degrees. The inclination angle is more preferably 18 to 40 degrees. The distance between the central portion at the leading end 42 of the inclined surface portion 4a and the central portion of the lumen 3 is preferably 10 to 40 µm. The distance is particularly preferably 15 to 35 µm. The distance between the central portion at the leading end 42 of the inclined surface portion 4a and the upper end of the lumen 3 is preferably 7 to 35 µm. The distance is particularly preferably 10 to 30 µm.

The cell suction holding pipette according to the present invention may also include an inclined surface portion 4b shown in FIG. 10 to FIG. 12. As shown in FIGS. 10 to 12, in a cell suction holding pipette 1b of this embodiment, the inclined surface portion 4b includes a substantially triangular inclined surface 41 whose width increases toward the leading end of the hollow body 2, and further includes a steeply inclined leading end portion 44 that is formed at the leading end of the inclined surface portion 4b and inclines toward the leading end side extension line of the central axis of the lumen 3 at an inclination angle larger than the inclination angle of the other portion of the substantially triangular inclined surface portion 41. More specifically, the inclined surface portion 4b includes the substantially triangular inclined surface 41 whose width increases toward the leading end of the hollow body, and the steeply inclined leading end portion 44 extending from the leading end of the inclined surface 41 toward the leading end of the hollow body and inclining toward the leading end side extension line of the central axis of the lumen 3 at an inclination angle larger than the inclination angle of the substantially triangular inclined surface 41. The steeply inclined leading end portion 44 reaches the leading end 42 of the pipette. The only difference between the cell suction holding pipette 1b of this embodiment and the cell suction holding pipette 1 of the above embodiment is the difference in the shape of the inclined surface portion.

The axial length of the inclined surface portion 4b is preferably 40 to 160 µm, and particularly preferably 60 to 140 µm. The width (width in the direction orthogonal to the central axis of the pipette) of the steeply inclined leading end portion 44 is preferably 10 to 30 µm. The width is particularly preferably 15 to 25 µm.

As in the pipette 1 described above, in the cell suction holding pipette 1b of this embodiment as well, the inclination angle of the planar inclined surface (substantially triangular inclined surface) 41 with respect to the central axis of the lumen 3 is preferably 10 to 50 degrees. The inclination angle is particularly preferably 15 to 45 degrees. The inclination angle is more preferably 18 to 40 degrees. The distance between the central portion at the leading end 42 of the inclined surface portion 4b and the central portion of the lumen 3 is preferably 10 to 40 µm. The distance is particularly preferably 15 to 35 µm. The distance between the central portion at the leading end 42 of the inclined surface portion 4b and the upper end of the lumen 3 is preferably 7 to 35 µm. The distance is particularly preferably 10 to 30 µm. Further, the inclination angle of the steeply inclined leading end portion 44 with respect to the central axis of the lumen 3 is larger than the inclination angle of the planar inclined surface (substantially triangular inclined surface) 41 preferably by 3 to 15 degrees, and particularly preferably by 5 to 12 degrees.

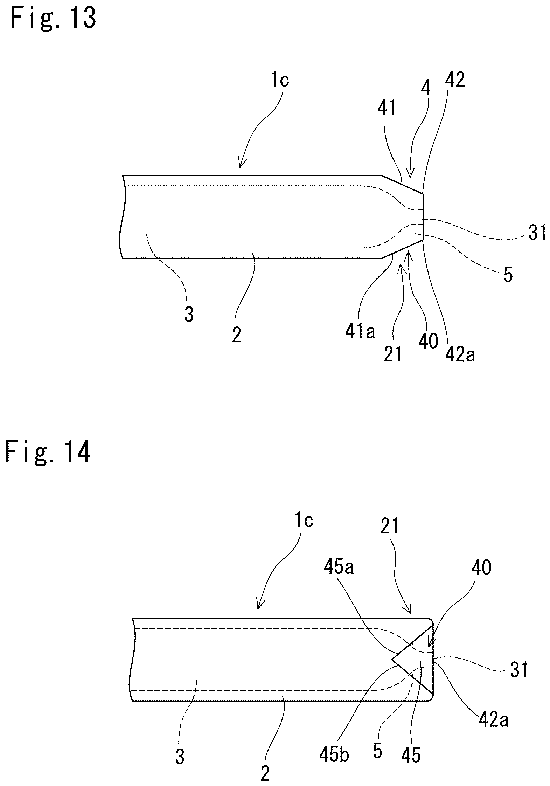

As shown in FIGS. 13 to 15, the cell suction holding pipette of the present invention may also include a plurality of inclined surface portions 4 and 40 inclining toward the leading end side extension line of the central axis of the lumen 3. A cell suction holding pipette 1c according to this embodiment includes two inclined surface portions 4 and 40. The two inclined surface portions 4 and 40 are opposed to each other with respect to the central axis of the lumen 3. The number of inclined surface portions may be two or more, and is preferably two or three.

The only difference between the cell suction holding pipette 1c of this embodiment and the cell suction holding pipette 1 of the above embodiment is the presence or absence of the second inclined surface portion 40. In the cell suction holding pipette 1c of this embodiment, the inclined surface portion 4 is similar to that of the cell suction holding pipette 1 of the above embodiment. Also, the second inclined surface portion 40 is the same as the inclined surface portion 4 described above.

More specifically, in the cell suction holding pipette 1c of this embodiment, the second inclined surface portion 40 includes a planar inclined surface 45 as shown in FIGS. 13, 14, and 15. Similarly to the inclined surface portion 4 described above, the planar inclined surface 45 is preferably a flat surface. However, the planar inclined surface 45 may be a gently curved surface. The curved surface may be an inwardly curved surface or an outwardly curved surface.

The second inclined surface portion 40 is preferably a flat surface. However, the planar inclined surface 45 of the inclined surface portion may have a shape whose central portion is recessed as in the cell suction holding pipette 1d of the embodiment shown in FIG. 16. Examples of the case where the planar inclined surface 45 has a shape whose central portion is recessed include a case where the planar inclined surface 45 slopes toward the center from both sides 45a and 45b and a case where the planar inclined surface 45 is gently curved inward.

In this embodiment, the planar inclined surface 45 is a substantially triangular inclined surface 45 extending to the leading end of the hollow body 2 with its width increasing toward the leading end. Preferably, the sides 45a and 45b of the planar inclined surface 45 have no edge, and are chamfered or rounded, for example. The axial length of the inclined surface portion 40 is preferably 40 to 160 µm, and particularly preferably 60 to 140 µm.

The planar inclined surface 45 reaches the leading end of the hollow body 2 and inclines at approximately the same angle. The inclination angle of the planar inclined surface 45 (see R1 in FIG. 4) with respect to the central axis of the lumen 3 is preferably 10 to 50 degrees. If the inclination angle is 10 degrees or more, it is possible to guide cells placed on the inclined surface portion 40 toward the leading end of the pipette, and if the inclination angle is 50 degrees or less, it is possible to restrict rapid movement of cells placed on the inclined surface portion 40. The inclination angle is particularly preferably 15 to 45 degrees. The inclination angle is more preferably 18 to 40 degrees. The distance between the central portion at the leading end 42a of the inclined surface portion 40 and the central portion of the lumen 3 (see D1 in FIG. 4) is preferably 10 to 40 µm. The distance is particularly preferably 15 to 35 µm. The distance between the central portion at the leading end 42a of the inclined surface portion 40 and the upper end of the lumen 3 (see D2 in FIG. 4) is preferably 7 to 35 µm. The distance is particularly preferably 10 to 30 µm.

In the cell suction holding pipette 1c according to this embodiment as well, the inclined surface portions 4 and 40 may have the shape of the inclined surface portion 4a of the cell suction holding pipette 1a shown in FIGS. 7 to 9 or the shape of the inclined surface portion 4b of the cell suction holding pipette 1b of the embodiment shown in FIGS. 10 to 12.

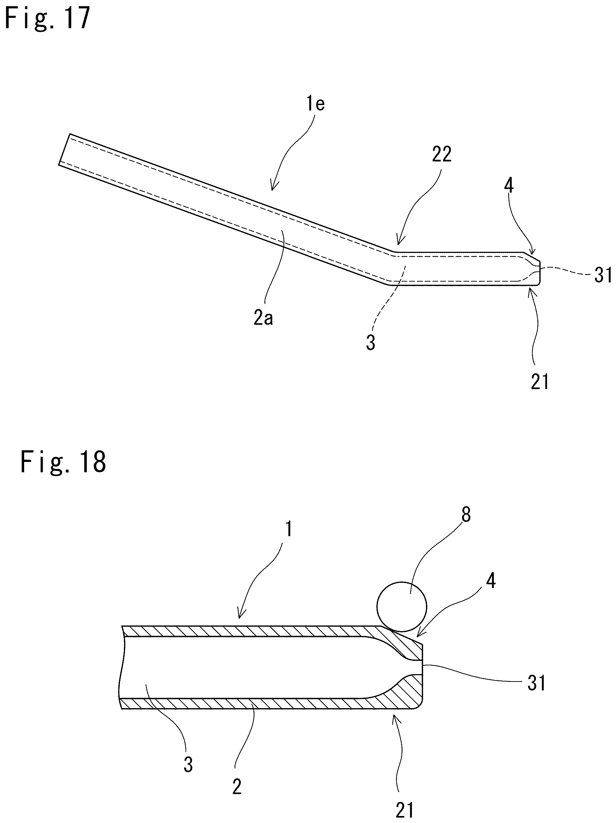

Furthermore, in the cell suction holding pipettes of all the embodiments described above, the hollow body may include a bent portion 22 as in the case of a hollow body 2a of a cell suction holding pipette 1e of an embodiment shown in FIG. 17. In this embodiment, the bent portion 22 is provided at a position closer to the leading end than a central portion of the hollow body 2a is. Also, the bending angle of the bent portion 22 with respect to the central axis of the lumen 3 is preferably 10 to 50 degrees, and particularly preferably 15 to 45 degrees. Furthermore, in this embodiment, the inclined surface portion 4 is provided in the leading end portion 21 of the hollow body 2a on the inner side in the bending direction of the bent portion 22.

Next, the following describes a cell suction holding operation performed using the cell suction holding pipette 1 according to the present invention with reference to FIGS. 18 to 20.

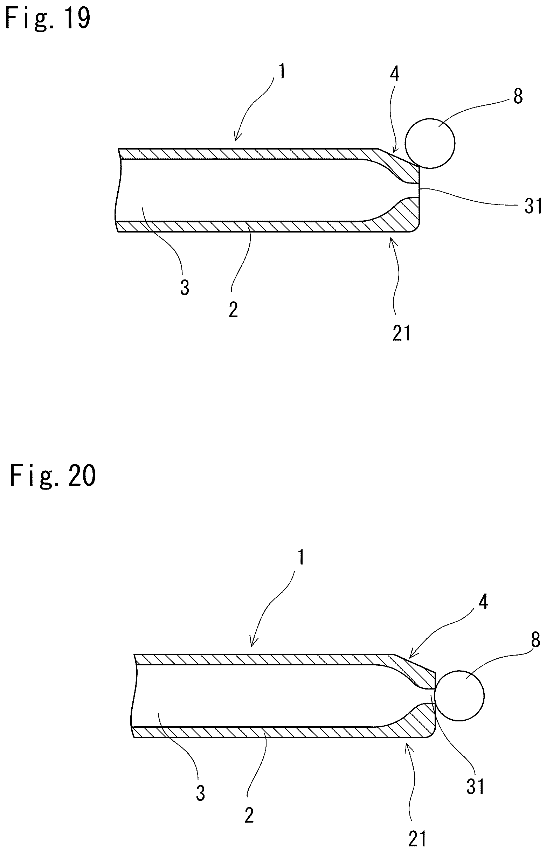

First, cells are collected by suction using another pipette. Then, the leading end portion of the cell suction holding pipette 1 is inserted into a laboratory dish (not shown) containing a culture solution. Subsequently, a leading end of the pipette into which the cells have been collected by suction is disposed above the inclined surface portion 4 of the cell suction holding pipette 1 and a cell 8 is discharged onto the inclined surface portion 4 as shown in FIG. 18. Then, a suction means (not shown) connected to a rear end portion of the cell suction holding pipette 1 is actuated. The discharged cell 8 moves along the inclined surface portion 4 toward the leading end and downward as shown in FIG. 19. Then, as shown in FIG. 20, the cell 8 that has come to a position below the leading end 42 of the inclined surface portion 4 is suctioned and held by the leading end opening 31 of the cell suction holding pipette 1.

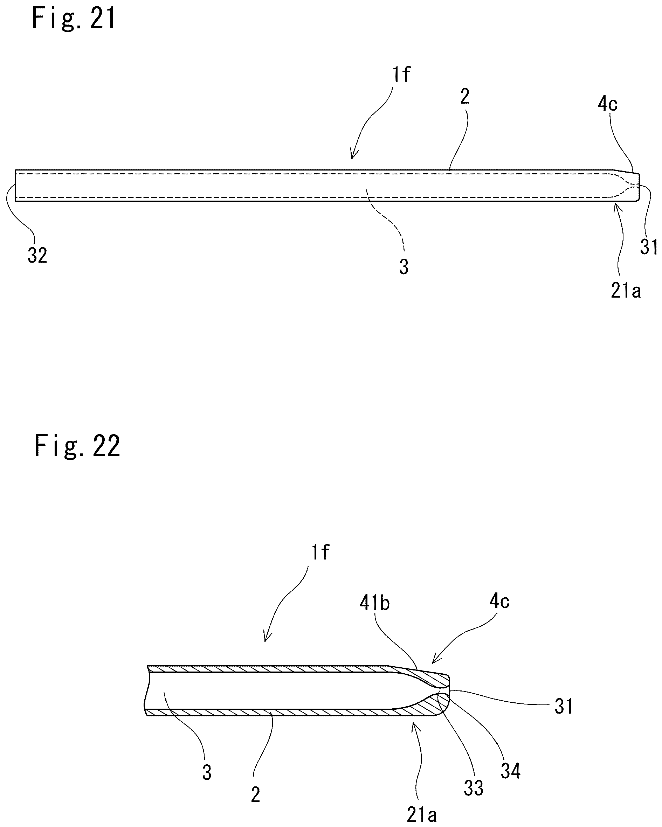

The following describes a cell suction holding pipette according to another embodiment of the present invention with reference to FIGS. 21 and 22.

Similarly to the cell suction holding pipette 1 described above, a cell suction holding pipette 1f of this embodiment includes a hollow body 2 and a lumen 3 having a leading end opening 31 and a rear end opening 32. The hollow body 2 includes a leading end portion 21a, and the leading end portion 21a includes an inclined surface portion 4c that is formed on an outer surface of the leading end portion 21a and inclines toward the leading end side extension line of the central axis of the lumen 3.

The basic structure of the cell suction holding pipette 1f of this embodiment is the same as that of the cell suction holding pipette 1 described above except that the shape of the leading end portion 21a is slightly different.

In this embodiment as well, a straight tube having substantially the same outer diameter and extending linearly is used as the hollow body 2. The material of the hollow body 2 is as described above regarding the cell suction holding pipette 1.

In the cell suction holding pipette 1f of this embodiment, the lumen 3 includes a leading end small inner diameter portion, and the leading end portion 21a of the hollow body 2 includes a thick leading end portion 5. Therefore, the inner diameter of the lumen 3 in the hollow body 2 is not constant, but becomes smaller at the leading end portion.

More specifically, as shown in FIG. 22, in the cell suction holding pipette 1f, the inner diameter of the lumen 3 sharply decreases in the leading end portion 21a. Accordingly, the leading end portion 21a of the cell suction holding pipette 1f includes the thick portion 5 having an annular shape. The inner diameter (opening diameter) of the leading end opening 31 of the lumen 3 is smaller than the inner diameter (opening diameter) of the rear end opening 32.

The cell suction holding pipette 1f of this embodiment also includes a leading end lumen 33 that is in communication with the lumen 3 and has a small inner diameter in the leading end portion 21a. A tapered inner diameter reduced portion in which the inner diameter sharply decreases from the lumen 3 side toward the leading end lumen 33 is formed at a base end of the leading end lumen 33. Also, a tapered inner diameter increased portion 34 in which the inner diameter gradually increases toward the leading end opening 31 is formed at a leading end of the leading end lumen 33.

Similarly to the cell suction holding pipette 1 described above, the outer diameter of the hollow body 2 (outer diameter of the leading end of the hollow body) of the cell suction holding pipette 1f is preferably 70 to 140 µm, and particularly preferably 80 to 130 µm. The inner diameter (opening diameter) of the leading end opening 31 is preferably 10 to 40 µm, and particularly preferably 12 to 35 µm.

Similarly to the cell suction holding pipette 1 described above, when the outer diameter of the hollow body 2 (outer diameter of the leading end of the hollow body) of the cell suction holding pipette 1f is 70 to 110 µm, the inner diameter (opening diameter) of the leading end opening 31 is preferably 10 to 20 µm, and particularly preferably 12 to 18 µm. When the outer diameter of the hollow body 2 (outer diameter of the leading end of the hollow body) of the cell suction holding pipette 1f is 110 to 140 µm, the inner diameter (opening diameter) of the leading end opening 31 is preferably 20 to 40 µm, and particularly preferably 25 to 35 µm.

The inner diameter of a region of the leading end lumen 33 having the smallest inner diameter is smaller than the inner diameter (opening diameter) of the leading end opening 31 preferably by 0.5 to 5 µm, and particularly preferably by 0.8 to 3 µm. The entire length of the leading end lumen 33 (the length from the leading end opening 31 to a region in the leading end lumen 33 having the same inner diameter as the leading end opening) is preferably 20 to 50 µm, and particularly preferably 25 to 40 µm. The length of the tapered inner diameter increased portion 34 is preferably 10 to 40 µm, and particularly preferably 15 to 35 µm.

Similarly to the cell suction holding pipette 1 described above, the cell suction holding pipette 1f includes the inclined surface portion 4c that is formed on the outer surface of the leading end portion 21a of the hollow body 2 and inclines toward the leading end side extension line of the central axis of the lumen 3. As described above, the lumen 3 includes the leading end small inner diameter portion, and the leading end portion 21a of the hollow body 2 includes the thick leading end portion 5. Accordingly, in this embodiment, the inclined surface portion 4c is formed on the outer surface of the thick leading end portion 5. Therefore, the leading end portion 21a of the hollow body 2 is not weakened due to the inclined surface portion 4c, and has sufficient strength.

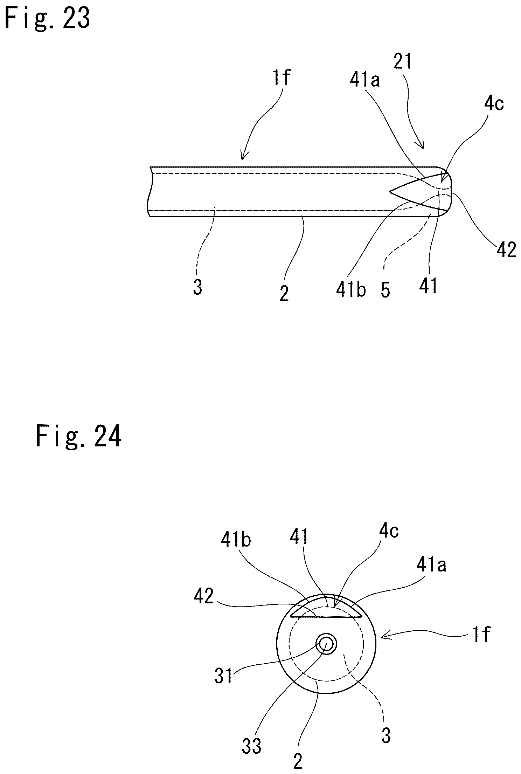

Similarly to the cell suction holding pipette 1 shown in FIGS. 2, 5, and 6, in the cell suction holding pipette 1f of this embodiment as well, the inclined surface portion 4c includes a planar inclined surface 41 as shown in FIGS. 22 to 24. The inclined surface portion is preferably a flat surface.

In this embodiment, the planar inclined surface 41 is a substantially triangular inclined surface 41 extending to the leading end of the hollow body 2 with its width increasing toward the leading end. Preferably, sides 41a and 41b of the planar inclined surface 41 have no edge, and are chamfered or rounded, for example. The axial length of the inclined surface portion 4c is preferably 40 to 160 µm, and particularly preferably 60 to 140 µm. Also, the axial length of the inclined surface portion 4c may be 60 to 200 µm. The axial length is particularly preferably 80 to 180 µm. Although the lateral width of a leading end portion of the inclined surface portion 4c varies depending on the outer diameter of the end portion of the pipette, the lateral width is preferably 65 to 120 µm, and particularly preferably 70 to 100 µm. Also, the lateral width of the leading end portion of the inclined surface portion 4c is preferably 50/100 to 80/100, and particularly preferably 60/100 to 75/100 of the outer diameter of the end portion of the pipette.

Similarly to the cell suction holding pipette 1 described above, in the cell suction holding pipette 1f of this embodiment as well, the planar inclined surface 41 reaches the leading end of the hollow body 2 and inclines at approximately the same angle as shown in FIGS. 22 to 24. The inclination angle (see R1 in FIG. 4) of the planar inclined surface 41 with respect to the central axis of the lumen 3 is preferably 5 to 30 degrees. If the inclination angle is 5 degrees or more, it is possible to slowly guide cells placed on the inclined surface portion 4c toward the leading end of the pipette. The inclination angle is particularly preferably 5 to 25 degrees.

The distance between a central portion at the leading end 42 of the inclined surface portion 4c and the central portion of the lumen 3 (see D1 in FIG. 4) is preferably 10 to 40 µm. The distance is particularly preferably 15 to 35 µm. The distance between the central portion at the leading end 42 of the inclined surface portion 4c and the upper end of the lumen 3 (see D2 in FIG. 4) is preferably 7 to 35 µm. The distance is particularly preferably 10 to 30 µm.

Similarly to the cell suction holding pipette 1a shown in FIGS. 7 to 9, in the cell suction holding pipette 1f of this embodiment as well, the inclined surface portion may include a flat leading end portion 43 that is formed at the leading end of the inclined surface portion and extends substantially in parallel with the central axis of the lumen 3. In this case, the inclined surface portion includes the substantially triangular inclined surface 41 whose width increases toward the leading end of the hollow body, and the flat leading end portion 43 extending from the leading end of the inclined surface 41 toward the leading end of the hollow body substantially in parallel with the central axis of the lumen 3, and the flat leading end portion 43 reaches the leading end 42 of the pipette. The width (width in the direction orthogonal to the central axis of the pipette) of the flat leading end portion 43 is preferably 10 to 30 µm. The width is particularly preferably 15 to 25 µm.

Similarly to the cell suction holding pipette 1c shown in FIGS. 13 to 15, the cell suction holding pipette 1f of this embodiment may include a plurality of (more specifically, two) inclined surface portions 4 inclining toward the leading end side extension line of the central axis of the lumen 3. The two inclined surface portions are opposed to each other with respect to the central axis of the lumen 3. It should be noted that the number of inclined surface portions may be two or more, and is preferably two or three.

Next, the following describes a cell suction collecting operation performed using a device for collecting cells from a fertilized egg or an embryo according to the present invention.

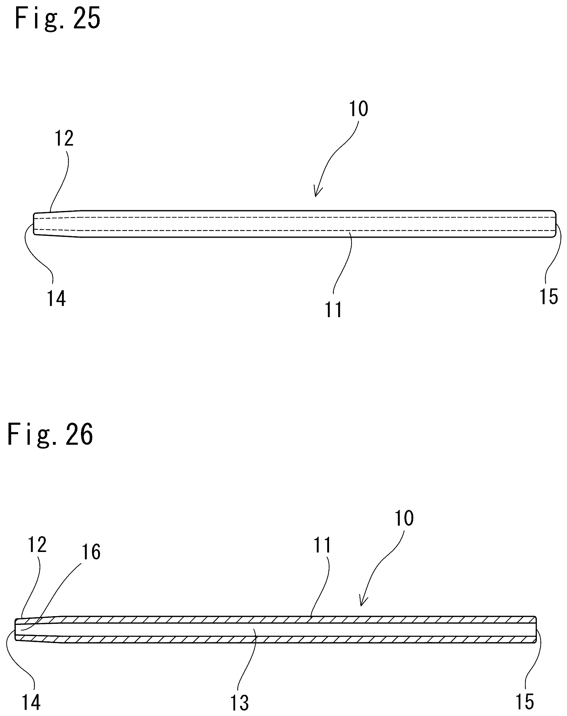

The device for collecting cells from a fertilized egg or an embryo according to the present invention includes the cell suction holding pipette described above and a cell suction collecting pipette 10. As shown in FIGS. 25 and 26, the cell suction collecting pipette 10 is a hollow tubular member including a leading end portion whose outer diameter is smaller than the outer diameter of the leading end portion of the cell suction holding pipette, and includes a hollow body 11, a leading end opening 14, a base end opening 15, and a lumen 13 formed in the hollow body 11. The leading end side portion of the lumen 13 serves as a cell collecting portion 16. A leading end portion 12 of the cell suction collecting pipette 10 of this embodiment includes a diameter reduced portion that is tapered toward the leading end of the cell suction collecting pipette 10.

The cell suction collecting pipette is for collecting part of cells from a fertilized egg or embryo (desirably, an embryo) by suction.

The cell suction collecting pipette 10 of an embodiment shown in FIGS. 25 and 26 is a hollow tubular member including the hollow body 11, the lumen 13 formed in the hollow body, the leading end portion 12, the leading end opening 14, the base end opening 15, and the cell collecting portion 16 formed in the leading end portion 12 (leading end side portion of the lumen 13).

In this embodiment, a straight tube having substantially the same outer diameter except for the leading end portion and extending linearly is used as the hollow body 11. It is also possible to use a cell suction collecting pipette that does not include the tapered diameter reduced portion at the leading end portion.

The material of the hollow body 11 is glass or a hard synthetic resin, and is preferably transparent. Examples of the glass include glass containing silicate as a main component (silicate glass). Examples of silicate glass include soda lime glass, borosilicate glass, and quartz glass. The glass may contain components other than silicate as a main component. Examples of such glass include acrylic glass, chalcogenide glass, metallic glass, and organic glass. In particular, borosilicate glass is preferable because alkaline elution can be suppressed. Examples of the hard synthetic resin include polyolefins such as high-density polyethylene (HDPE) and polypropylene (PP), polystyrene, polyethylene terephthalate, and polycarbonate.

As shown in FIGS. 25 and 26, the leading end portion 12 of the cell suction collecting pipette 10 includes the diameter reduced portion that is tapered toward the leading end of the cell suction collecting pipette 10. In this embodiment, both the outer diameter and the inner diameter of the leading end portion 12 of the cell suction collecting pipette 10 decrease toward the leading end of the cell suction collecting pipette 10. The outer diameter of the leading end portion of the cell suction collecting pipette 10 is smaller than the outer diameter of the leading end portion of the cell suction holding pipette used.

The outer diameter of the leading end of the leading end portion 12 (outer diameter of the leading end of the hollow body 11) of the cell suction collecting pipette 10 is preferably 30 to 120 µm, and particularly preferably 40 to 90 µm. Furthermore, the outer diameter of the leading end of the leading end portion 12 of the cell suction collecting pipette 10 is smaller than the outer diameter of the leading end portion of the cell suction holding pipette used preferably by 20 to 100 µm, and particularly preferably by 30 to 85 µm.

Furthermore, the gradient angle (taper angle) of the diameter reduced portion of the leading end portion 12 of the cell suction collecting pipette 10 is smaller than the inclination angle of the substantially triangular inclined surface of the cell suction holding pipette with respect to the central axis of the lumen. The gradient angle (taper angle) of the diameter reduced portion of the leading end portion 12 of the cell suction collecting pipette 10 is preferably 3 to 20 degrees, and particularly preferably 5 to 10 degrees. The gradient angle (taper angle) of the diameter reduced portion of the leading end portion 12 of the cell suction collecting pipette 10 is smaller than the inclination angle of the substantially triangular inclined surface of the cell suction holding pipette with respect to the central axis of the lumen preferably by 2 to 10 degrees, and particularly preferably by 3 to 8 degrees. Furthermore, the axial length of the diameter reduced portion of the leading end portion 12 of the cell suction collecting pipette 10 is preferably 40 to 200 µm, and particularly preferably 60 to 180 µm.

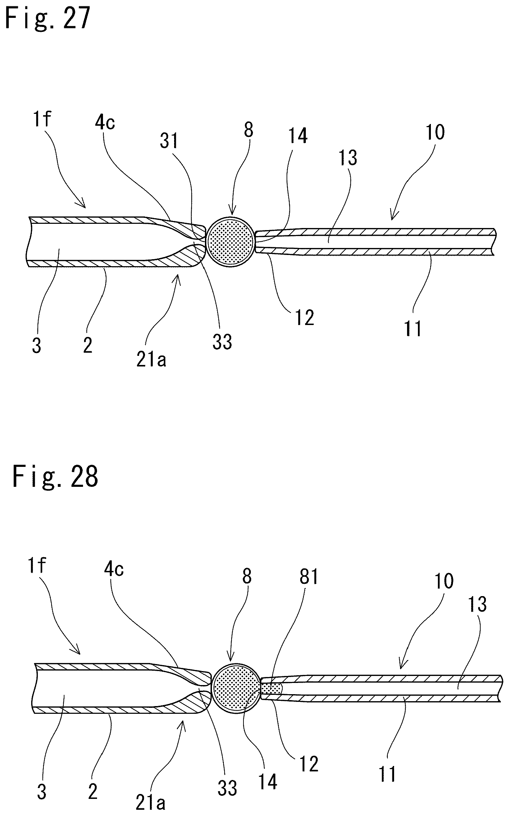

Next, the following describes the cell suction collecting operation (separation and collection of cells by suction) performed using the device for collecting cells from a fertilized egg or an embryo according to the present invention with reference to FIGS. 27 to 31.

First, the cell suction holding pipette 1f suctioning and holding a fertilized egg or an embryo (preferably, an embryo) at its leading end opening 31 is prepared as shown in FIG. 20. Then, as shown in FIG. 27, the leading end opening 14 of the leading end portion 12 of the cell suction collecting pipette 10 is brought into contact with the embryo 8 held by the cell suction holding pipette 1f to sandwich the embryo 8 between the leading end of the cell suction holding pipette 1f and the leading end of the cell suction collecting pipette 10. Then, a suction means (not shown) connected to the rear end of the cell suction collecting pipette 10 is actuated to suction and collect part of cells from the inside of the embryo into the leading end portion 12 (cell collecting portion 16) of the cell suction collecting pipette 10 as shown in FIG. 28.

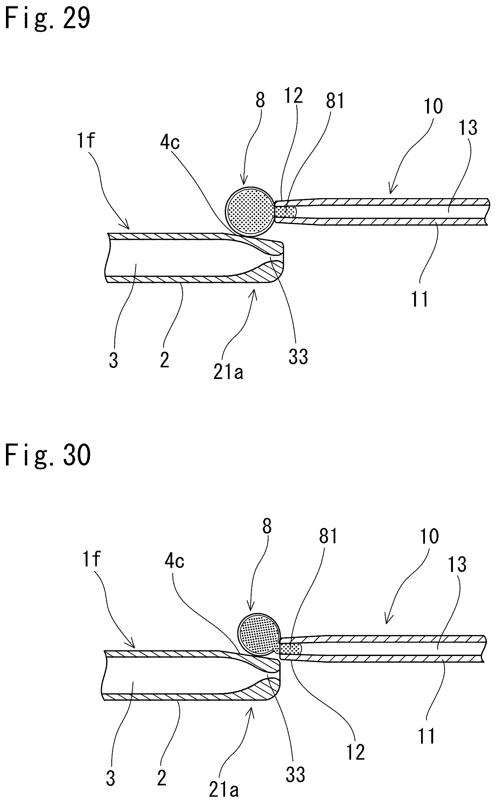

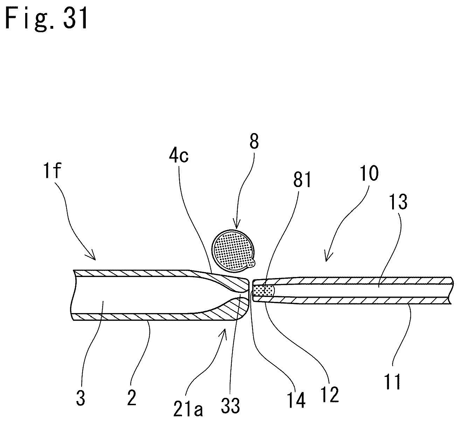

Subsequently, after the suction of the embryo 8 by the cell suction holding pipette 1f is ceased, the embryo 8 is kept suctioned and held by the cell suction collecting pipette 10, and the embryo 8 is positioned on the inclined surface portion 4c of the cell suction holding pipette 1f as shown in FIG. 29. Then, as shown in FIG. 30, the cell suction collecting pipette 10 is operated to move the embryo 8 toward the leading end on the inclined surface portion 4c, and a lower portion of the leading end of the cell suction collecting pipette 10 is positioned above the leading end of the cell suction holding pipette 1f. Subsequently, the lower portion of the leading end of the cell suction collecting pipette 10 is moved toward a lower portion of the leading end of the cell suction holding pipette 1f. As a result, the embryo 8 is partially broken (partially separated) as shown in FIG. 31, and part of the cells of the embryo 8 is collected in the leading end portion (cell collecting portion 16) of the cell suction collecting pipette 10.

The thus obtained part of the cells of the embryo can be used for a cell test, specifically, for a preimplantation genetic testing for aneuploidy for analyzing genes.

### Industrial Applicability

A cell suction holding pipette of the present invention is as follows.
(1) A cell suction holding pipette comprises a hollow body and a lumen having a leading end opening and a rear end opening, wherein the hollow body includes a leading end portion, and the leading end portion includes an inclined surface portion formed on an outer surface of the leading end portion and inclining toward a leading end side extension line of a central axis of the lumen.

This cell suction holding pipette includes the hollow body and the lumen having the leading end opening and the rear end opening, the hollow body includes the leading end portion, and the leading end portion includes the inclined surface portion that is formed on the outer surface of the leading end portion and inclines toward the leading end side extension line of the central axis of the lumen.

Therefore, when a cell collected into a collecting pipette is discharged onto the inclined surface portion of the cell suction holding pipette, the discharged cell is guided by the inclined surface portion toward the leading end of the cell suction holding pipette, and then moves downward from the leading end toward a leading end surface of the cell suction holding pipette. The cell that has moved downward is suctioned and held by the leading end opening of the pipette by a suction force acting on the cell suction holding pipette. Thus, it is possible to easily perform the operation for suctioning and holding cells.

The above embodiment may also be as follows.
(2) The cell suction holding pipette of the above (1), wherein the lumen includes a leading end small inner diameter portion, the leading end portion of the hollow body includes a thick leading end portion, and the inclined surface portion is formed on an outer surface of the thick leading end portion.
(3) The cell suction holding pipette of the above (1) or (2), wherein the inclined surface portion includes a substantially triangular inclined surface whose width increases toward a leading end of the hollow body.
(4) The cell suction holding pipette of the above (3), wherein the substantially triangular inclined surface is a flat surface and has an inclination angle of 10 to 50 degrees with respect to the central axis of the lumen.
(5) The cell suction holding pipette of the above (4), wherein the substantially triangular inclined surface inclines at approximately the same angle up to the leading end of the hollow body.
(6) The cell suction holding pipette of the above (1) or (2), wherein the inclined surface portion includes a substantially triangular inclined surface whose width increases toward a leading end of the hollow body, and further includes a flat leading end portion formed at a leading end portion of the inclined surface portion and extending substantially in parallel with the central axis of the lumen.
(7) The cell suction holding pipette of the above (1) or (2), wherein the inclined surface portion includes a substantially triangular inclined surface whose width increases toward a leading end of the hollow body, and further includes a steeply inclined leading end portion formed at a leading end portion of the inclined surface portion and inclining toward the leading end side extension line of the central axis of the lumen at an inclination angle larger than an inclination angle of another portion of the substantially triangular inclined surface.

A device for collecting cells from a fertilized egg or an embryo according to the present invention is as follows.
(8) A device for collecting cells from a fertilized egg or an embryo, the device comprising: the cell suction holding pipette according to any one of the above (1) to (7); and a cell suction collecting pipette, wherein the cell suction collecting pipette is a hollow tubular member including a leading end portion whose outer diameter is smaller than an outer diameter of the leading end portion of the cell suction holding pipette, the cell suction collecting pipette includes a hollow body, a leading end opening, a base end opening, and a lumen formed in the hollow body, and a leading end side portion of the lumen serves as a cell collecting portion.

This device for collecting cells makes it possible to easily perform an operation for separating a fertilized egg or an embryo and collected cells suctioned into the leading end portion of the cell suction collecting pipette by using the leading end portion of the cell suction collecting pipette and the inclined surface portion and the leading end portion of the cell suction holding pipette.

The above embodiment may also be as follows.
(9) The device for collecting cells from a fertilized egg or an embryo of the above (8), wherein the leading end portion of the cell suction collecting pipette is a diameter reduced portion that is tapered toward a leading end.
(10) The device for collecting cells from a fertilized egg or an embryo of the above (9), wherein the inclined surface portion of the cell suction holding pipette includes a substantially triangular inclined surface whose width increases toward a leading end of the hollow body, and
   a gradient angle of the diameter reduced portion in the leading end portion of the cell suction collecting pipette is smaller than an inclination angle of the substantially triangular inclined surface of the cell suction holding pipette with respect to the central axis of the lumen.

## Claims

1. A cell suction holding pipette comprising:
a hollow body; and
a lumen having a leading end opening and a rear end opening,
wherein the hollow body includes a leading end portion, and the leading end portion includes an inclined surface portion formed on an outer surface of the leading end portion and inclining toward a leading end side extension line of a central axis of the lumen.

2. The cell suction holding pipette according to claim 1,
wherein the lumen includes a leading end small inner diameter portion, the leading end portion of the hollow body includes a thick leading end portion, and the inclined surface portion is formed on an outer surface of the thick leading end portion.

3. The cell suction holding pipette according to claim 1 or 2,
wherein the inclined surface portion includes a substantially triangular inclined surface whose width increases toward a leading end of the hollow body.

4. The cell suction holding pipette according to claim 3,
wherein the substantially triangular inclined surface is a flat surface and has an inclination angle of 10 to 50 degrees with respect to the central axis of the lumen.

5. The cell suction holding pipette according to claim 4,
wherein the substantially triangular inclined surface inclines at approximately the same angle up to the leading end of the hollow body.

6. The cell suction holding pipette according to claim 1 or 2,
wherein the inclined surface portion includes a substantially triangular inclined surface whose width increases toward a leading end of the hollow body, and further includes a flat leading end portion formed at a leading end portion of the inclined surface portion and extending substantially in parallel with the central axis of the lumen.

7. The cell suction holding pipette according to claim 1 or 2,
wherein the inclined surface portion includes a substantially triangular inclined surface whose width increases toward a leading end of the hollow body, and further includes a steeply inclined leading end portion formed at a leading end portion of the inclined surface portion and inclining toward the leading end side extension line of the central axis of the lumen at an inclination angle larger than an inclination angle of another portion of the substantially triangular inclined surface.

8. A device for collecting cells from a fertilized egg or an embryo, the device comprising:
the cell suction holding pipette according to any one of claims 1 to 7; and
a cell suction collecting pipette,
wherein the cell suction collecting pipette is a hollow tubular member including a leading end portion whose outer diameter is smaller than an outer diameter of the leading end portion of the cell suction holding pipette,
the cell suction collecting pipette includes a hollow body, a leading end opening, a base end opening, and a lumen formed in the hollow body, and
a leading end side portion of the lumen serves as a cell collecting portion.

9. The device for collecting cells from a fertilized egg or an embryo according to claim 8,
wherein the leading end portion of the cell suction collecting pipette is a diameter reduced portion that is tapered toward a leading end.

10. The device for collecting cells from a fertilized egg or an embryo according to claim 9,
wherein the inclined surface portion of the cell suction holding pipette includes a substantially triangular inclined surface whose width increases toward a leading end of the hollow body, and
a gradient angle of the diameter reduced portion in the leading end portion of the cell suction collecting pipette is smaller than an inclination angle of the substantially triangular inclined surface of the cell suction holding pipette with respect to the central axis of the lumen.
